# EUROPEAN PATENT APPLICATION

(11) **EP 3 236 247 A1**
(43) Date of publication of application: **25.10.2017**
(21) Application number: 16165973.5
(22) Date of filing: 19.04.2016
(51) Int. Cl.: G01N 25/48, G01N 33/44

(54) **METHOD FOR DETERMINING CURING DEGREE OF A RESIN AND USE THEREOF**

(71) Applicant: Borealis Agrolinz Melamine GmbH, 4021 Linz (AT)
(72) Inventor: PUCHINGER, Helmut, 4240 Freistadt (AT); DICKE, Rene, 4060 Leonding (AT)
(74) Representative: Maikowski & Ninnemann Patentanwälte Partnerschaft mbB

(57) **Abstract**

The present invention relates to a method for determining the degree of curing of a resin wherein the resin to be analyzed comprises the resin by itself, the resin mixed with at least one particle or fiber containing material and/or the resin applied to at least one particle or fiber containing material, wherein the degree of resin curing is determined using differential scanning calorimetry (DSC) and a pressure between 20 and 500 bar, preferably between 30 and 300 bar, in particular preferably between 50 and 150 bar, and wherein the pressure is applied to DSC measuring cell containing the sample to be analyzed before starting the DSC measurement. The present invention relates also to the use of such method for optimizing process parameters for example in wood board production.

## Description

The present invention relates to a method for determining the degree of curing of a resin according to the preamble of claim 1 and the use of said method according to claim 12.

### Description

Non-coated wood based panels like chipboards or particle boards (PB), OSB, medium density fiber (MDF), low density fiber (LDF) or high density fiber (HDF) are made of wood particles or wood fibers. In the course of the production process of such boards the particles or fibers are mixed with an adhesive, the mixture is arranged on conveyers, which are guided through a series of presses (pre, hot presses). There the adhesive is cured and in the curing process strong bonds between the wood particles or wood fibers and the adhesive are created. The boards obtained can be used in different areas, but are predominantly used as carrier boards for floor laminates.

Thus, in the woodworking industry - especially for the laminate producers - it is very important to identify the right amount of curing agent for curing the adhesive, such as melamine formaldehyde resins. The curing mechanism of melamine formaldehyde resins depends strongly on the curing time, the curing temperature and the amount/type of the curing agent. Also the melamine : formaldehyde ratio has an impact on the curing behavior, as well as the melamine quality itself and the use of resin additives.

Different methods for analyzing the curing behavior of resins have been described in the literature.

Kandelbauer et al. (Interscience, Wiley, 2009) investigated the curing kinetics of a liquid melamine-formaldehyde impregnation resin with differential scanning calorimetry, and the conversion dependent activation energy for melamine-formaldehyde resin curing was estimated with the isoconversional model-free kinetic approach developed by Vyazovkin (Thermochim. Acta, 1999, 340-341, 53-68). The conversion-dependent activation energy was used to extend the predictive power of a response surface model describing the influence of some processing parameters (press time and resin composition) in the manufacturing of particle boards coated with melamine-formaldehyde-impregnated papers.

Vyazovkin's recommendations offer guidance for reliable evaluation of kinetic parameters (activation energy, the pre-exponential factor and the reaction model) from data obtained by means of thermal analysis methods such as thermogravimetry (TGA), differential scanning calorimetry (DSC) and differential thermal analysis (DTA). The recommendations cover the most common kinetic methods, model-free (isoconversional) as well as model-fitting.

Vázquez et al. (Journal of Thermal Analysis and Calorimetry, 2005, Vol. 82, 143-149) investigated the curing behavior of phenol-urea-formaldehyde-tannin (PUFT) adhesives via kinetic studies. PUFT adhesives have been prepared by copolymerization at room temperature of pine bark tannins with PUF pre-polymers under different operating conditions. DSC calorimetry and DMA have been used to analyze the curing of pre-polymers and adhesives. DSC curves at three different heating rates were obtained by means of the model free kinetics method; chemical conversion versus time at a given temperature was obtained. Mechanical conversion was calculated from DMA storage modulus data for those adhesives which gave the best results for plywood and MDF boards.

The laboratory methods or quality control methods of manufacturers applied for determining the curing agent content are typically conducted by applying a temperature profile at different heating rates. However, the water content in the resin is not sufficiently considered thus providing results which do not resemble the actual process conditions for the industrial wood based panel production.

In particular, each laminate or particle board producer has his own subjective method to determine the amount/type of curing agent which also means that there is no standardized method at the moment.

Thus, there is a need to provide a reliable and standardized method for determining the curing behavior and curing kinetics of a resin used in the wood panel production process which reflects the actual process conditions.

The object of the present invention is thus to provide a method that can be used for determining the curing kinetics of a resin under real conditions for the industrial wood based panel production.

This object is solved by a method according to claim 1 and the use of such a method according to claim 12.

Accordingly, a method for determining the degree of curing (i.e. curing behavior or curing kinetics) of a resin is provided
- wherein the resin to be analyzed comprises the resin, the resin mixed with at least one particle or fiber containing material and/or the resin applied to at least one particle or fiber containing material,
- wherein the degree of resin curing is determined using differential scanning calorimetry (DSC) and a pressure between 20 and 500 bar, preferably between 30 and 300 bar, in particular preferably between 50 and 150 bar,
- wherein the pressure is applied to a DSC measuring cell containing the sample to be analyzed before starting the DSC measurement.

In a preferred embodiment of the present method the DSC measurement is conducted by applying a predetermined temperature profile (or temperature scan) at different heating rates. The temperature profile ranges from 100 to 300 °C, preferably from 130 to 250 °C, most preferably from 170 to 230 °C. The heating rates are in a range between 1 and 50 K/min, preferably between 2 and 30 K/min, most preferably between 5 and 20 K/min. For example, heating rates may be 2, 5, 10, 15 or 20 K/min.

In a further preferred embodiment of the present method the pressure is not formed in situ in the DSC measuring cell. A pressure build up in the DSC measuring cell usually occurs due to water or solvent evaporation that is present in the sample to be analyzed. In contrast according to the present method the pressure is applied for suppressing the endothermic step of water evaporation.

Furthermore, the present method simulates process conditions as applied in the production process of wood particle boards and/or laminates. Thus, the present method provides experiments and experimental results that are closer to the production conditions at the customer's site. The present method based on a pressure DSC allows first time a prediction of the curing behavior under nearly real process conditions. This allows for synchronizing or aligning process conditions and amount of curing agent / hardener outside of the production line (offline) to the actual production line.

The present method allows for predicting or determining the required amount for a selected curing agent for curing a resin, such as amino resins especially melamine formaldehyde and urea formaldehyde resins and/or a mixture of the two resin systems. With the present invention it is possible to predict curing time of a resin with different amounts and types of curing agents for defined curing temperatures.

When conducting the high pressure DSC experiments according to the present method the endothermic water peak during the curing cycle of the polycondensation reaction of the melamine formaldehyde or urea formaldehyde resins in the DSC thermogram can be suppressed by applying pressures for example > 100 bars. Using the measured DSC curves a TAK (thermal analysis kinetics) was applied to identify the kinetic model for the curing reaction of melamine formaldehyde resin curing. Based on the calculated model a prediction of conversion to the final product (e.g. cured resin) system can be made for defined temperatures. The calculated data are applicable in case the processing time (curing time in pressing step of the lamination process) in lamination process is the criteria for comparison.

The present method allows for the determination of the curing agent type under real process conditions. It is possible to obtain curing information for several curing agent contents at different curing temperatures. Besides it is easy to obtain fast curing information if a resin system is changed during the production process.

The advantages of the present method can be summarized as follows:
- DSC measurements done under high pressure therefor no pre-drying step is needed;
- Measurements and result interpretation done at nearly real process conditions in the DSC;
- Standardized method for curing agent type determination;
- Standardized method for determination of the ideal amount of curing agent;
- Determination of the optimal conversion degree providing optimal curing time at a defined curing temperature; this allows for a maximum product performance and prediction of the conversion degree fitting to maximum product performance;
- Determination of the optimal conversion degree providing optimal curing time at a defined curing temperature; this allows for a maximum production speed for coated or non-coated wood based panels;
- Determination of pH-dependency during resin synthesis for curing in particular in case the resin formulations are changed in the production cycle;
- Determination of resin quality, in particular melamine quality used in a resin that influences curing;
- Determination of influences of resin additives on curing behavior of resin;
- Determination of formaldehyde content of resins on curing behavior of resin;
- Selection of an appropriate curing agent without the requirement of testing the curing agent in the production process.

In a further preferred embodiment of the present method the resin to be analyzed comprises at least one curing agent. In particular, it is preferred if varying types and amounts of at least one curing agent are added to the resin to be analyzed. When applying the present method the amounts and types of curing agent and curing times are varied. In particular, the determination of the preferred curing agent is done at a conversion degree between 70- 100%, preferably between 90-98%. Thus, the present method allows for a standardized method for determination of the type and amount of curing agent

The curing agent is an acid or an acidic salt, such as an ammonium salt, in particular ammonium nitrate, ammonium sulfate, ammonium chloride ammonium sulfonate, and/or morpholinyl salt, in particular morpholinyl toluene sulfonate.

In a further embodiment of the present method the amount of the at least one curing agents is between 0.05 and 2 wt%, preferably between 0.1 and 1 wt%.

In an embodiment of the present method the resin to be analyzed is a formaldehyde resin, in particular a melamine-formaldehyde resin, a urea-formaldehyde-resin, melamine-urea-formaldehyde resin, phenol-formaldehyde resin or a mixture thereof.

In a further preferred embodiment of the present method the particle or fiber containing material is to be understood that the particles or fibers are used in a loose manner (for example loose wood fibers) or in an integrated / bound manner (for example wood fibers pressed in a wood panel).

The particles or fibers may be selected from a group comprising wood particles, such as wood chips, wood splinters, wood strands, wood fibers, cellulose fibers (for example non-bleached or bleached), hemp fibers, cotton fibers or any other type of plant fibers or synthetic fibers. In this case it is furthermore preferred that the (loose) mixture comprising the resin and the at least one fiber material is a homogenous or heterogeneous mixture.

The particle or fiber containing material may also be selected from a group comprising paper (as cellulose containing material), in particular decor paper, core paper, Kraft paper or overlay paper (as for example used in the production of laminate or for decoration of other wood based boards). Said papers or paper layers are typically impregnated with said resin. The particle or fiber containing material may be also selected from wood fiber panels, such as LDF, MDF; HDF plywood panels or OSB boards.

The resin may be applied to the particle or fiber containing material; i.e. the resin may be applied as surface coating (forming a resin layer on the material) or may be used for impregnation. In the latter case the resin penetrates into the material, such as a paper layer.

In case the present method is used for determining the curing degree of a resin applied to a particle or fiber containing material (such as decor paper) a sample of said material is taken and crushed or shredded such that the sample can be filled into the DSC measuring cell.

It is also possible that the particle / fiber material with the resin comprises further additives, such as wetting agents, flexibility modifier, antiseize agents, release agent, coupling agents, fillers, pigments, plasticizers, stabilizers, anti-static additives or flame retardants.

In the present method the degree of resin curing is determined at temperatures between 100 and 300 °C, preferably between 130 and 250 °C, in particular preferably between 170 and 230 °C.

The present method can be applied for optimizing process parameters in wood based panel production, wherein a resin is used as adhesive, cover or protective layer and/or as impregnating agent. In particular, the present method is used for optimizing type and amount of curing agent added to the resin and curing time.

In one variant the present method is used for determining the curing behavior of a resin that is used as adhesive for fiber material, in particular wood fibers or strands, in the production of coated or non-coated wood based boards, in particular particle boards, LDF panels, HDF board, MDF boards, ply wood, OSB boards.

One possible application of the present method is exemplarily described by means of particle board or plywood production.

The production process of particle boards or panels, such as particle boards starts by providing wood particles, such as wood chips or wood splinters,' that are dried and subsequently mixed (for instance by spraying) with an appropriate amount of adhesives, in particular melamine-formaldehyde-resin, urea-formaldehyde-resin or phenol-formaldehyde-resin. A curing agent is added to the adhesive or resin before spraying on the wood particles. The mixture of wood particles, adhesive and curing agent is then placed on a conveyer that is passed through a series of hot presses for pre-pressing and final pressing.

The final properties of the particle board are substantially influenced by the type and amount of curing agent used in the adhesive mixture. This determines the speed of the press in order to obtain the best possible quality or balance between curing or hardening in the press and final conditioning. This requires that the appropriate amount of curing agent and adhesive is used with the corresponding pressing temperature and pressing time.

Conventionally, in order to determine the appropriate amount of curing agent the adhesive and resin were mixed and the appropriate amount of curing agent was determined under lab conditions. The established lab methods are based on a measurement at temperatures of about 100 °C. This, however, does not correspond to real pressing temperatures in the production process where temperatures between 180 and 240 °C are applied. Due to the different temperatures between lab method and industrial process the lab results have to be converted to industrial process conditions based on experience and empirical data. For example, a curing time of 3-4 min at 100 °C for a resin in the lab corresponds to 10-20 sec at 200 °C depending on the board thickness in the industrial process line.

The present method now allows to determine the amount of curing agent, pressing temperature, pressing time under nearly real process conditions. The present method allows for fixing the parameter pressing temperature and thus to reduce the number of variables. Thereby, the pressing temperature may be defined in any desired manner, and the parameters curing agent and pressing time can be determined, accordingly.

The present method allows for a direct and immediate transfer of the results obtained by the present method to the production process since the variable pressing temperature can be fixed in the temperature range relevant for the industrial pressing step. This opens the possibility for a more precise adjustment of the complete production line, in particular in order to use optimal amounts of curing agent (for cost reduction and best possible mechanical performance of the wood board); to apply optimal pressing times for a prefixed amount of curing agent and to influence the final properties of the wood boards.

In another embodiment of the present method the resin is used as impregnating agent for paper sheets and/or adhesive in the production of laminates.

In the production process of laminates a resin solution is prepared comprising typically about 50wt% water. The paper sheet to be impregnated is passed through the resin solution in a series of impregnating baths followed by a pre-drying step at a temperature profile up to 150 °C. The pre-drying induces a pre-curing that depends strongly on the curing system since preferably thermo-latent curing agents are used which are activated only at a certain temperature. The water content of such an impregnated paper is typically reduced in the process to about 6-10wt%.

In the subsequent steps the impregnated paper is either pressed onto a wood board (such as a fiber board or a plywood board) or onto so called core-papers or kraft-papers. In this pressing step a temperature of about 180-240 °C is applied.

In the described lamination process the present method can be applied using the impregnating solution comprising the curing agent by itself and/or in addition the paper sheet impregnated with the resin.

The present invention is explained in more detail by means of the following examples with reference to the figures. It shows:
- Figure 1: DSC curves for MF resins without curing agent at different pressures (white curve - 1 bar, red curve - 100 bar with a heating rate of 10 K/min);
- Figure 2: DSC curves for a resin with 0,2 wt% curing agent with different heating rates [20 K/min upper curve, 10 K/min, 5 K/min, 2 K/min red curve below];
- Figure3: Conversion degree as a function of time for different curing temperatures [curing agent content in the resin formulation 0.0 wt%, max. curing time 0.1 min];
- Figure 4: Conversion degree as a function of time for different curing temperatures [curing agent content in the resin formulation 0.2 wt%, max. curing time 0.1 min];
- Figure 5: Conversion degree as a function of time for different curing temperatures [curing agent content in the resin formulation 0.4 wt%, max. curing time 0.1 min],
- Figure 6: DSC thermogram of impregnated paper at three different heating rates;
- Figure 7: a diagram showing curing of impregnated paper as a function of curing time at different curing temperatures;

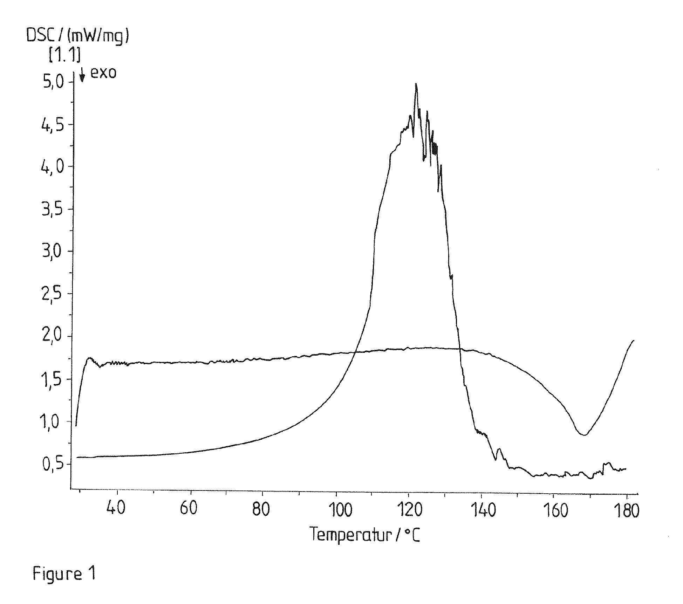

The change of the physical state (liquid water soluble resin - into a solid crosslinked network) can be determined by running DSC measurements. The curing of these liquid resins is in the first step an endothermic reaction (evaporation of the water) which can be obtained from DSC experiments, followed by an exothermic curing step of the resin. Target of this investigation was the usage of high pressure DSC to suppress the endothermic step. By running the experiments under high pressure the evaporation of the water can be eliminated. This effect is shown in figure 1. Further, the high pressure mimics the industrial conditions of curing the resins in a press.

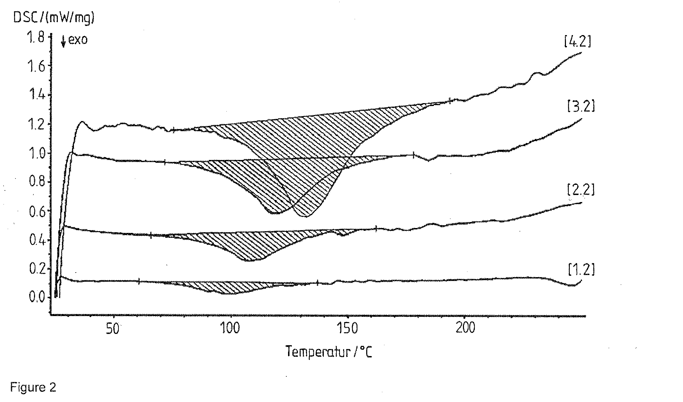

Using a temperature controlled program for DSC, the crosslinking reaction of the resins was tracked. The heating curves at different heating rates are shown in fig. 2. The DSC curves referenced with 1.2, 2.2, 3.2 and 4.2 correspond to heating rates of 2.0, 5.0, 10.0 and 20.0 K/min, respectively. All curves show the same exothermic peak area.

Fig. 2 shows the shifting of the curves to higher temperatures with higher heating rates. This shift reflects only the equipment limits, because the higher the heating rate is the slower the equipment can show the exothermic changes.

The purpose of thermal analysis kinetics (TAK) is to quantitatively characterize reaction processes. In the current work TAK was used to characterize the crosslinking reaction of MF resin curing to obtain probable mechanism function f(alpha), the kinetic parameters of the activation energy E and the pre-exponential factor A, the rate constant and the expression of the reaction rate from simulated curves. The herein used mathematic models are also described in the state closed art, and can be explained if needed for the present invention.

Finally with the model-fit method a relationship between α, E and A is established. First a reaction model is selected which more or less fits the measured data, the parameters are optimized by multi-variant non-linear regression to obtain a precise model which makes it feasible to describe the crosslinking degree as a function of temperature and curing time for a certain amount of curing agent.

The theoretically calculated relationships between conversion and reaction time under isothermal conditions for a resin comprising a curing agent amount of 0.2 wt% are shown in Table 1. Curing of the resin at 130 °C with a curing time of approximately one minute leads to a conversion to the final product of approximately 60 % compared to a curing temperature of 140 °C where the conversion of approximately 80 % is achieved. Here a conversion model of the curing process for higher temperatures is depicted which equals shorter reaction time to reach full conversion. A conversion higher than 90% can be obtained after approximately 0.1 min for a temperature of 200 °C.

More precisely the conversion of the resin to the final cured product is shown in table 1. For a curing temperature of 200 °C 0.2 minutes are need to reach a conversation degree of 95%. It must be mentioned here that this curing times are similar to curing times for final product curing in an industrial process which indicates that the model describes the reality in a sufficient way.

**Table 1: Time needed to reach a certain conversation for different curing temperatures for a resin with 0.2 wt% of curing agent**

| TIME VERSUS FINAL PRODUCT | | | | | | |
|---|---|---|---|---|---|---|
| Max. Time/min: 10.0 | | | | | | |
| | Temperature/°C | | | | | |
| Final Product/% | 170.0 | 180.0 | 190.0 | 200.0 | 210.0 | 220.0 |
| 2 | 4.5E-3 | 2.8E-3 | 1.8E-3 | 1.1E-3 | 7.9E-4 | 5.3E-4 |
| 5 | 1.0E-2 | 6.8E-3 | 4.4E-3 | 2.8E-3 | 1.9E-3 | 1.2E-3 |
| 10 | 2.1E-2 | 1.3E-2 | 8.3E-3 | 5.4E-3 | 3.6E-3 | 2.4E-3 |
| 15 | 3.0E-2 | 1.9E-2 | 1.2E-2 | 7.8E-3 | 5.2E-3 | 3.5E-3 |
| 20 | 3.9E-2 | 2.4E-2 | 1.5E-2 | 1.0E-2 | 6.7E-3 | 4.5E-3 |
| 25 | 4.7E-2 | 3.0E-2 | 1.9E-2 | 1.2E-2 | 8.2E-3 | 5.6E-3 |
| 30 | 5.6E-2 | 3.5E-2 | 2.3E-2 | 1.4E-2 | 9.8E-3 | 6.6E-3 |
| 35 | 6.6E-2 | 4.1E-2 | 2.6E-2 | 1.7E-2 | 1.1E-2 | 7.7E-3 |
| 40 | 7.6E-2 | 4.7E-2 | 3.0E-2 | 2.0E-2 | 1.3E-2 | 8.9E-3 |
| 45 | 8.6E-2 | 5.4E-2 | 3.5E-2 | 2.3E-2 | 1.5E-2 | 1.0E-2 |
| 50 | 9.8E-2 | 6.1E-2 | 3.9E-2 | 2.6E-2 | 1.7E-2 | 1.1E-2 |
| 55 | 0.111 | 7.0E-2 | 4.5E-2 | 2.9E-2 | 1.9E-2 | 1.3E-2 |
| 60 | 0.126 | 7.9E-2 | 5.1E-2 | 3.3E-2 | 2.2E-2 | 1.4E-2 |
| 65 | 0.145 | 9.1E-2 | 5.8E-2 | 3.8E-2 | 2.5E-2 | 1.7E-2 |
| 70 | 0.168 | 0.105 | 6.7E-2 | 4.4E-2 | 2.9E-2 | 2.0E-2 |
| 75 | 0.198 | 0.124 | 8.0E-2 | 5.2E-2 | 3.4E-2 | 2.3E-2 |
| 80 | 0.241 | 0.151 | 9.7E-2 | 6.3E-2 | 4.2E-2 | 2.8E-2 |
| 85 | 0.307 | 0.193 | 0.123 | 8.0E-2 | 5.3E-2 | 3.6E-2 |
| 90 | 0.432 | 0.271 | 0.173 | 0.113 | 7.5E-2 | 5.1E-2 |
| 95 | 0.777 | 0.487 | 0.312 | 0.203 | 0.135 | 9.1E-2 |
| 98 | 1.738 | 1.090 | 0.697 | 0.455 | 0.302 | 0.204 |

In the past the determination of the curing time, the curing temperature and the determination of the ideal amount of curing agent for curing of MF-resins was not straightforward. Several methods (determination of the clouding time of resins, determination of the gel time) for exact curing agent amount were implemented. However, these methods have the disadvantage of its determination at 100 °C, while curing of MF resins occurs in reality at 180 to 220 °C.

If curing was done via the former method at 100 °C the beginning of curing was detected after 264 seconds, end of curing at 310 seconds. It is obvious that these values are far away from reality.

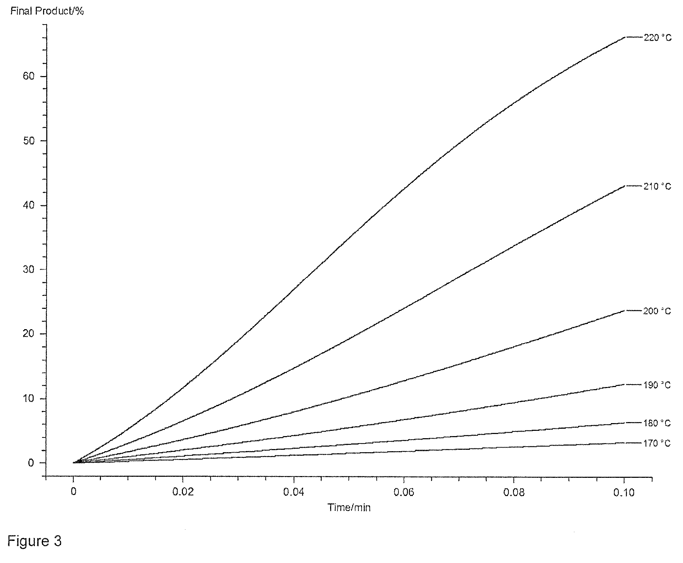

Target of the method is as well to determine the right amount of curing agent for the process. In a first step the resin curing is accelerated enormous by adding a curing agent. This effect can be shown by comparing fig. 3-5. The method allows the prediction of the curing at nearly real industrial conditions.

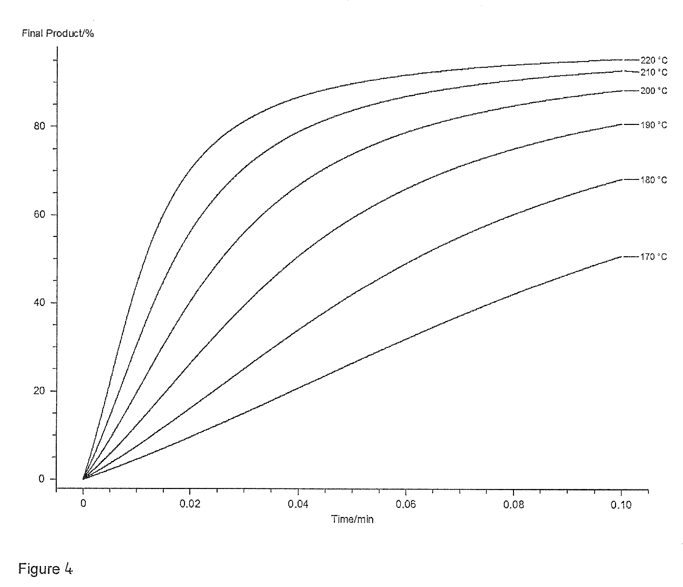

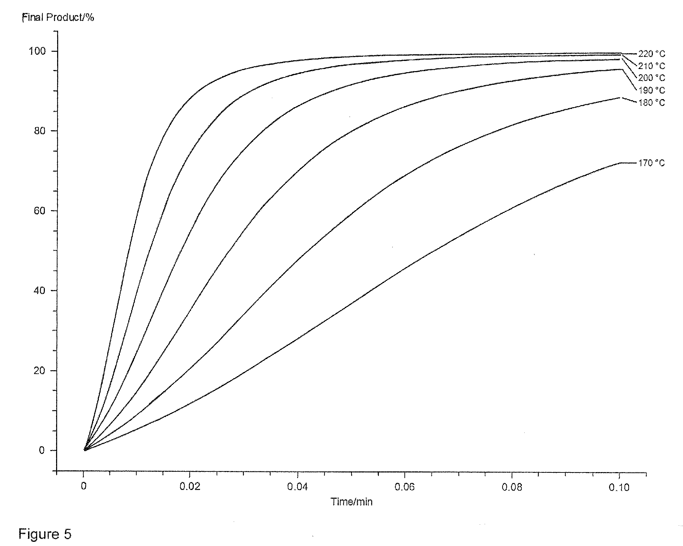

The curing agent has a huge impact to the curing of the resin. When no curing agent is used for the curing process, the curing cycle takes much more time (fig. 3). Only by adding 0.2 wt% (fig. 4) or 0.4wt% (fig.5) of curing agent the curing process is accelerated tremendously. If further curing agent is added the curing process is accelerated however not in that amounts compared by adding curing agent in a first step. By adding even more curing agent the curing process is not accelerated more. However, a too high amount of curing agent can affect the final properties of a coated or non-coated boards negatively.

Furthermore, the amount of curing agent has a significant impact on the curing velocity especially at higher temperatures. For example, when looking at the curing behavior of a resin with 0%, 0.3%, 0.35 %, 0.4 % and 0.45 % at 100 °C, 190 °C and 200 °C, respectively, the curing velocity increases with increasing curing agent concentration. Interestingly, the velocity increase is not the same at 100 °C, 190 °C and 200 °C. At 100 °C there is rather a small difference in velocity increase at different curing agent concentrations, whereas at 190 °C and 200 °C this difference is more concise. For example, at 100 °C and 200 sec curing time the resin conversion using 0.3% curing agent was about 30% and using 0.4% curing agent was about 40%. At 190 °C and 5 sec curing time the resin conversion using 0.3% curing agent was about 45% and using 0.4% curing agent was about 85%. At 200 °C and 5 sec curing time the resin conversion using 0.3% curing agent was about 60% and using 0.4% curing agent was about 90%.

Thus, the present method allows to select an appropriate curing agent without the requirement of testing the curing agent in the production process.

### Example 1: Resin

### a) Resin synthesis:

The chosen recipe for this evaluation is a standard MF resin. 434.4 g Formaldehyde solution (36%), 47.4 g diethylene glycol and 113.9 g water are charged into the reactor. The temperature of the mixture is adjusted to about 25 °C. After adding 404.3 g of the melamine, the pH-value is adjusted with diluted sodium hydroxide solution (0.1 N) to a value of 8.8±0.2. The solution is heated up to 93 °C as fast as possible. After reaching the target temperature, the mixture is heated for a short period to 95-98 °C, to dissolve the melamine faster. Immediately after all melamine is dissolved, the temperature is decreased to 93 °C, at which the condensation procedure is done up to a water tolerance (wₜ) of 2, which is an indicator for the condensation rate. That means the cooling down starts after this water tolerance (vol.water/vol.resin) is reached.

### b) DSC experiments:

DSC was performed on a Netzsch STA 449 C Jupiter. The heating rates were 2, 5, 10 and 20 K/min in an N₂ atmosphere. The temperature range was from 25 °C to 250 °C by applying a pressure of 100 bar.

Approximately 10 g of resin solution was mixed with different amounts of curing agent Deurocure KS - (0,2, 0,4, 0,6 and 0,8 wt %). As a reference, the unmodified resin was also investigated in the DSC equipment.

### Example 2: Impregnated decor paper for laminate production

Decor papers of conventional laminates are usually impregnated with a melamine-formaldehyde-resin (MF resin according to Example 1) with a melamine : formaldehyde ratio of 1.6-1.8. Said laminates have to pass a test requires according to DIN EN 438 before being traded.

The manually impregnated papers are measured regarding their curing behavior by DSC. Subsequently, the curing kinetics of said papers is determined by TAK.

The impregnating resin solution is obtained by adding 0.5 % of a release agent (corresponds to 2g), and 0.5 % wetting agent (corresponds to 2g) to 400 g resin solution.

A curing agent is added to one part of the impregnating resin solution, whereas the other part of the impregnating resin solution contains no curing agent.

Viscosity and clouding times are determined of both solutions: viscosity 270 mPas, clouding time 90 sec for the resin solution with curing agent.

A decor paper is provided and manually impregnated using the impregnating device at the following conditions: singular impregnation, smooth scraper, drying for 90 min in air and subsequently for 2 min at 120 °C in a drying cabinet. One paper sheet is dried for further 10 min at 160 °C in a drying cabinet.

The resin application, resin content and residual moisture of decor papers were determined.

**Table 2: parameter of impregnated decor paper**

| | curing agent content x% |
|---|---|
| Total amount of Resin applied to the paper | 215.4 % |
| Amount of Resin in the paper | 68.3 % |
| Residual moisture | 8.9 % |

### Determination of curing kinetics of the impregnated paper:

DSC measurements were performed for the impregnated paper and a raw paper (as reference without resin impregnation). The impregnated paper was cut in small pieces and placed in the DSC device. The heating rates were 10, 15, 20 K/min in a N₂ atmosphere. The temperature range was from 25 °C to 250 °C by applying a pressure of 100 bar.

In a DSC curve of impregnated paper (not shown) taken at a heating rate of 15 K/min exothermic curing peaks between 80 and 170 °C are detectable.

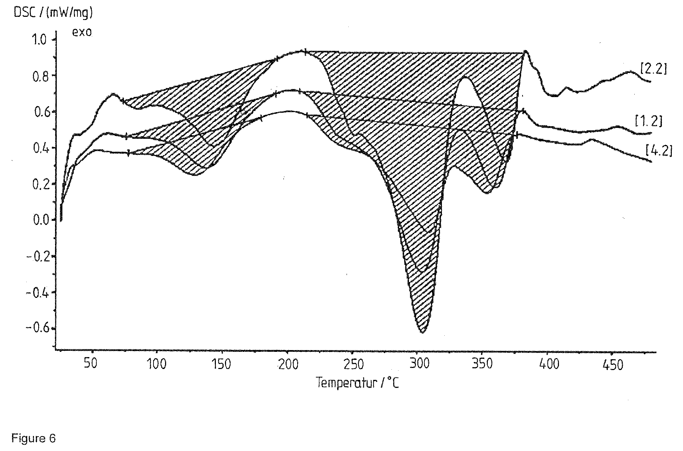

Fig. 6 shows DSC thermograms of impregnated papers at three different heating rates (10, 15, 20 K/min). The curves with the reference signs 4.2, 1.2 and 2.2 correspond to heating rates 10, 15 and 20 K/min, respectively.

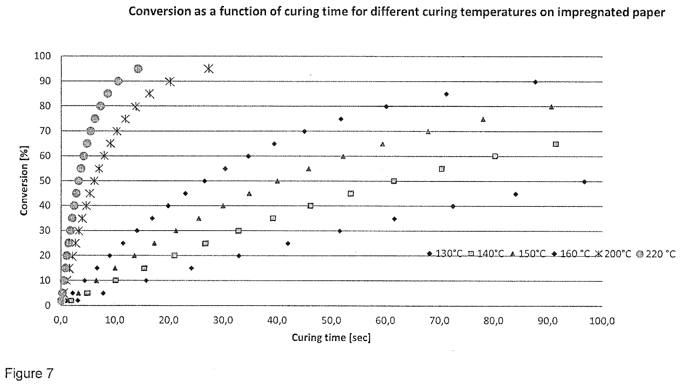

The diagram of Fig. 7 illustrates the curing behavior of impregnated paper as a function of time and temperature. As deducible the reduction of curing time corresponds to an increase of temperatures.

## Claims

1. Method for determining the degree of curing of a resin
- wherein the resin to be analyzed comprises the resin, the resin mixed with at least one particle or fiber containing material and/or the resin applied to at least one particle or fiber containing material,
- wherein the degree of resin curing is determined using differential scanning calorimetry (DSC) and a pressure between 20 and 500 bar, preferably between 30 and 300 bar, in particular preferably between 50 and 150 bar,
- wherein the pressure is applied to DSC measuring cell containing the sample to be analyzed before starting the DSC measurement.

2. Method according claim 1, **characterized in that** the DSC measurement is conducted by applying a predetermined temperature profile at different heating rates.

3. Method according to claim 1 or 2, **characterized in that** the DSC measurement is conducted in a temperature profile ranging from 100 to 300 °C, preferably from 130 to 250 °C, most preferably from 170 to 230 °C at heating rates ranging between 1 and 50 K/min, preferably between 2 and 30 K/min, most preferably between 5 and 20 K/min.

4. Method according to one of the preceding claims, **characterized in that** the pressure is not formed in situ in the DSC measuring cell.

5. Method according to one of the preceding claims, **characterized in that** the resin to be analyzed comprises at least one curing agent.

6. Method according to claim 5, **characterized in that** the amount of the at least one curing agents is between 0.05 and 2 wt%, preferably between 0.1 and 1 wt%,

7. Method according to one the preceding claims, **characterized in that** the resin to be analyzed is a formaldehyde resin, in particular a melamine-formaldehyde resin, a urea-formaldehyde-resin, melamine-urea-formaldehyde resin, phenol-formaldehyde resin or a mixture thereof.

8. Method according to one of the preceding claims, **characterized in that** the resin comprises further additives, in particular wetting agents, flexibility modifier, antiseize agents, release agent, coupling agents, fillers, pigments, plasticizers, stabilizers, anti-static additives or flame retardants.

9. Method according to one of the preceding claims, **characterized in that** the particles or fibers are selected from a group comprising wood particles, such as wood chips, wood powders, wood splinters, wood strands, wood fibers, cellulose fibers, in particular non-bleached or bleached cellulose fibers, hemp fibers, cotton fibers or any other type of plant fibers or synthetic fibers.

10. Method according to one of the preceding claims, **characterized in that** the particle or fiber containing material is selected from a group comprising paper, in particular decor paper, core paper, Kraft paper or overlay paper, and wood fiber panels, in particular LDF, MDF; HDF plywood panels or OSB boards.

11. Method according to one of the preceding claims, **characterized in that** the degree of resin curing is determined at temperatures between 100 and 300 °C, preferably between 130 and 250 °C, in particular preferably between 170 and 230 °C.

12. Use of a method according to one of the preceding claims for optimizing process parameters in wood based board production, wherein a resin is used as adhesive, cover or protective layer and/or as impregnating agent.

13. Use according to claim 12 for optimizing type and amount of curing agent added to the resin and curing time.

14. Use according to claim 12 or 13, **characterized in that** the resin is used as adhesive for fiber material, in particular wood fibers or strands, in the production of coated and non-coated wood based boards, in particular particle boards, LDF boards, HDF board, MDF boards, ply wood, OSB boards.

15. Use according to claim 12 or 13, **characterized in that** the resin is used as impregnating agent for paper sheets and/or adhesive in the production of laminates.
